# FASCICULE DE BREVET EUROPEEN

(11) **EP 2 421 494 B1**
(45) Date de publication et mention de la délivrance du brevet: **07.02.2018**
(21) Numéro de dépôt: 10723693.7
(22) Date de dépôt: 16.04.2010
(51) Int. Cl.: A61K 8/02, A61K 8/88, A61Q 1/00, A61Q 1/14

(54) **PROCEDE DE FABRICATION DE PARTICULES DE POUDRE LIBRE A BASE DE POLYAMIDE IMPREGNEE D'AU MOINS UN AGENT COSMETIQUE OU PHARMACEUTIQUE, ET PARTICULES DE POUDRE LIBRE A BASE DE POLYAMIDE AYANT UNE TENEUR D'AU MOINS 25% EN POIDS D'AU MOINS UN AGENT COSMETIQUE OU PHARMACEUTIQUE**
VERFAHREN ZUR HERSTELLUNG VON FREIEN PULVERTEILCHEN VON POLYAMID, DIE MIT MINDESTENS EINEM KOSMETISCHEN ODER PHARMAZEUTISCHEN MITTEL IMPRÄGNIERT SIND, UND FREIE PULVERTEILCHEN VON POLYAMID MIT EINEM GEHALT VON MINDESTENS 25 GEW.-% EINES KOSMETISCHEN ODER PHARMAZEUTISCHEN MITTELS
METHOD FOR PRODUCING FREE POWDER PARTICLES OF POLYAMIDE IMPREGNATED WITH AT LEAST ONE COSMETIC OR PHARMACEUTICAL AGENT, AND FREE POWDER PARTICLES OF POLYAMIDE HAVING A CONTENT OF AT LEAST 25 WT % OF AT LEAST ONE COSMETIC OR PHARMACEUTICAL AGENT

(30) Priorité: 21.04.2009 FR 0952579
(43) Date de publication de la demande: 29.02.2012
(73) Titulaire: Arkema France, 92700 Colombes (FR)
(72) Inventeur: LOYEN, Karine, F-27500 Pont-audemer (FR)
(86) Numéro de dépôt international: PCT/FR2010/050740
(87) Numéro de publication internationale: WO 2010/122258

(56) Documents cités:
- EP-A1- 0 511 092
- EP-A1- 1 493 433
- EP-A1- 1 661 546
- EP-A1- 1 964 875
- WO-A1-03/022979
- WO-A1-2009/100962
- "Orgasol- cosmetic Ingredients", Arkema , 1 mars 2008 (2008-03-01), XP002568536, Extrait de l'Internet: URL:http://www.arkema.com/pdf/EN/products/ technical_polymers/orgasol/brochure_Orgaso l_cosmetics_2008.pdf [extrait le 2010-02-12]
- NACAPRICHA D ET AL: "Quality control of nuclear charcoals: particle size effect and trapping mechanism", CARBON, ELSEVIER, OXFORD, GB, vol. 34, no. 2, 1 January 1996 (1996-01-01), pages 155-163, XP004022364, ISSN: 0008-6223, DOI: 10.1016/0008-6223(96)00163-7

## Description

La présente invention concerne un procédé de fabrication de particules de poudre libre à base de polyamide imprégnée d'au moins un agent cosmétique ou pharmaceutique étant, à une température comprise dans la gamme de 15 à 25°C, sous la forme d'un liquide pur. La présente invention se rapporte notamment à des particules de poudre libre à base de polyamide ayant une teneur d'au moins 25% en poids d'au moins un agent cosmétique ou pharmaceutique autre que l'eau, ledit agent cosmétique ou pharmaceutique étant, à une température comprise dans la gamme de 15 à 25°C, sous la forme d'un liquide pur. L'invention se rapporte aussi à l'utilisation de ces particules à haute teneur en agent cosmétique ou pharmaceutique dans les produits cosmétiques, pharmaceutiques ou de parfumerie, et aux compostions cosmétiques, pharmaceutiques, ou de parfumerie comprenant de telles particules.

On connaît de nombreux exemples de compositions cosmétiques ou dermatologiques destinées au traitement de la peau, présentant un ou des actifs adaptés au traitement de la peau, encapsulés dans des microsphères de phospholipides ou des vésicules lipidiques (appelées aussi liposomes) ou dans des microsphères de polymères décrites par exemple dans le document EP0375520. Les inconvénients de ces techniques d'encapsulation sont la difficulté à contrôler le processus de fabrication des microsphères et leur manque de stabilité pendant leur stockage ou lors de leur introduction dans des formulations cosmétiques. Généralement, la quantité d'actif(s) introduite lors de la fabrication des micorsphères diffère de la quantité (inférieure) qui subsiste dans les microsphères pendant leur stockage, et diffère encore de la quantité (encore inférieure) relarguée au niveau de la peau.

La demande de brevet EP1493433 décrit des particules poreuses comprenant au moins un composé actif. Ces particules sont obtenues par un procédé qui consiste à imprégner des particules poreuses avec au moins un composé actif préalablement solubilisé dans un solvant tel que l'acétone, l'ethanol, l'isopropanol, le dichlorométhane, l'acétate d'éthyle, puis à évaporer le solvant jusqu'à l'obtention d'une poudre sèche. Toutefois on retrouve toujours une proportion même faible de ces solvants dans les poudres obtenues, et dans les produits finis qui incorporent ces poudres. Or ces solvants sont à l'origine d'effets indésirables sur la peau, tels que sécheresse, allergies, pigmentation, etc.

La brochure commerciale « Orgasol® Poudre polyamide ultra fine » datant de février 1991, et la gamme ORGASOL IMPREGNES décrite dans la revue « Cosmetics and Toiletries », volume 108, parue en décembre 1993 antériorisent la demande de brevet précitée. La brochure et la revue décrivent notamment l'imprégnation d'Orgasol® (poudre poreuse de polyamide 12) par un actif cosmétique solubilisé dans un solvant qui est ensuite généralement évaporé. Le taux final d'agent cosmétique ou pharmaceutique dans ces poudres imprégnées n'excède pas 20%.

La présente invention a donc pour but de fournir un procédé de fabrication de particules de poudre libre hautement chargées en agents cosmétiques ou pharmaceutiques, qui ne présente pas les inconvénients de l'art antérieur, qui évite la présence de solvants nocifs pour la peau, qui présente le moins d'étapes possibles, et qui n'altère pas les propriétés cosmétiques ou pharmaceutiques de ces agents.

La présente invention a également pour but de fournir des particules de poudre libre hautement chargées en agents cosmétiques ou pharmaceutiques, dont la quantité d'agents reste stable pendant le stockage des particules, lors de leur incorporation dans une formulation, et dont la diffusion de ces agents dans le stratum corneum de la peau est améliorée, c'est-à-dire augmentée et/ou prolongée après application topique de ces particules sur la peau. On entend par « particules de poudre libre hautement chargées en agent(s) cosmétique(s) ou pharmaceutique(s) » des particules ayant une teneur d'au moins 25% en poids, de préférence au moins 40% en poids, de cet (ou ces) agent(s).

On a maintenant montré qu'il était possible de fabriquer de telles particules de poudre libre hautement chargées en agents cosmétiques ou pharmaceutiques grâce à un nouveau procédé d'imprégnation de particules poreuses.

Par l'expression « particules poreuses », on désigne des particules comportant des pores. La porosité est caractérisée quantitativement par la surface spécifique (appelée aussi SSA). Les particules poreuses de l'invention présentent une SSA mesurée selon la méthode BET supérieure ou égale à 8m²/g. La méthode BET (BRUNAUER-EMMET-TELLER) est une méthode bien connue de l'homme du métier. Elle est notamment décrite dans « The journal of the American Chemical Society », vol.60, page 309, février 1938 et correspond à la norme internationale ISO 5794/1 (annexe D). La surface spécifique mesurée selon la méthode BET correspond à la surface spécifique totale, c'est-à-dire qu'elle inclut la surface formée par les pores.

Par l'expression « particules de poudre libre », on désigne des particules qui ne sont pas regroupées sous la forme d'agrégat ou d'agglomérat.

La présente invention a donc pour objet un procédé de fabrication de particules de poudre libre à base de polyamide imprégnée d'au moins un agent cosmétique ou pharmaceutique étant, à une température comprise dans la gamme de 15 à 25°C, sous la forme d'un liquide pur, ledit procédé étant constitué des étapes successives suivantes :
- ajout goutte à goutte ou par pulvérisation dudit agent cosmétique ou pharmaceutique à l'état liquide à des particules de poudre libre sous agitation, de sorte que l'agent cosmétique ou pharmaceutique représente au moins 25% en poids des particules de poudre, lesdites particules étant poreuses et de SSA supérieure à 8m²/g,
- arrêt de l'ajout avant que les particules de poudre ne commencent à s'agglomérer,
- maintien de l'agitation pendant au moins 5 minutes, de préférence au moins 20 minutes, puis
- récupération de la poudre libre obtenue imprégnée dudit agent cosmétique ou pharmaceutique.
Avantageusement, lesdites particules de poudre libre ont un diamètre moyen en volume compris dans la gamme allant de 3 à 12 µm, de préférence de 5 à 10 µm.
Selon un mode de réalisation préféré de l'invention, lesdites particules à base de polyamide sont choisies parmi les particules de polyamide, de copolyamide, de copolyesteramide, et leurs mélanges.

Par particules à base de polyamide (homopolyamide ou copolyamide) au sens de l'invention on entend les produits de condensation des lactames, des aminoacides et/ou des diacides avec les diamines et, en règle générale, tout polymère formé par des motifs reliés entre eux par des groupes amides. Les particules selon l'invention peuvent également être issues de la copolymérisation de lactame(s) avec une ou plusieurs lactone(s) conduisant à un copolyesteramide comme décrit dans le brevet EP1172396.

Le terme « monomère » dans la présente description des copolyamides doit être pris au sens d' « unité répétitive ». Le cas où une unité répétitive du polyamide est constituée de l'association d'un diacide avec une diamine est particulier. On considère que c'est l'association d'une diamine et d'un diacide, c'est-à-dire le couple diamine.diacide (en quantité équimolaire), qui correspond au monomère. Ceci s'explique par le fait qu'individuellement, le diacide ou la diamine n'est qu'une unité structurale, qui ne suffit pas à elle seule à polymériser. Dans le cas où les particules selon l'invention comprenant au moins deux monomères différents, appelés «co-monomères», c'est à dire au moins un monomère et au moins un co-monomère (monomère différent du premier monomère), elles forment un copolymère tel qu'un copolyamide abrégé CoPA ou bien un coplyesteramide abrégé CoPEA.

A titre d'exemple de lactames, on peut citer ceux ayant de 3 à 12 atomes de carbone sur le cycle principal et pouvant être substitués. On peut citer par exemple le β,β-diméthylpropriolactame, le α,α-diméthylpropriolactame, l'amylolactame, le caprolactame, le capryllactame, l'oenantholactame, le 2-pyrrolidone et le lauryllactame.

A titre d'exemple de diacide (ou acide dicarboxylique), on peut citer les acides ayant entre 4 et 18 atomes de carbone. On peut citer par exemple, l'acide adipique, l'acide sébacique, l'acide azélaique, l'acide subérique, l'acide isophtalique, l'acide butanedioïque, l'acide 1,4 cyclohexyldicarboxylique, l'acide téréphtalique, le sel de sodium ou de lithium de l'acide sulphoisophtalique, les acides gras dimérisés(ces acides gras dimérisés ont une teneur en dimère d'au moins 98% et sont de préférence hydrogénés) et l'acide dodécanédioïque HOOC-(CH2)10-COOH.

A titre d'exemple de diamine, on peut citer les diamines aliphatiques ayant de 6 à 12 atomes, elle peut être arylique et/ou cyclique saturée. A titre d'exemples on peut citer l'hexaméthylènediamine, la pipérazine, la tetraméthylène diamine, l'octaméthylène diamine, la décaméthylène diamine, la dodécaméthylène diamine, le 1,5 diaminohexane, le 2,2,4-triméthyl-1,6-diamino-hexane, les polyols diamine, l'isophorone diamine (IPD), le méthyl pentaméthylènediamine (MPDM), la bis(aminocyclohéxyl) méthane (BACM), la bis(3-méthyl-4 aminocyclohéxyl) méthane (BMACM), la méthaxylyènediamine, le bis-p aminocyclohexylméthane et la triméthylhexaméthylène diamine.

A titre d'exemple d'aminoacide, on peut citer les alpha-oméga aminoacides, tels que les acides aminocaproïque, amino-7-heptanoïque, amino-11-undécanoïque, n-heptyl-11-aminoundécanoïque et amino-12-dodécanoïque.

A titre d'exemple de lactone, on peut citer la caprolactone, la valérolactone et la butyrolactone.

Le ou les monomère(s) préférentiellement utilisé(s) dans l'invention est ou sont choisi(s) parmi les lactames tels que par exemple le lauryllactame, le caprolactame, l'oenantholactame, le capryllactame ou leurs mélanges. De préférence, on utilise le lauryllactame seul ou en mélange avec le caprolactame.
De préférence, les particules à base de polyamide selon l'invention comprennent, avant imprégnation, une teneur en pourcentage molaire de polyamide 12 comprise dans la gamme allant de 50% à 100%, de préférence de 80% à 100%.
De préférence, lesdites particules de poudre à base de polyamide sont obtenues au moins en partie par polymérisation anionique, par ensemencement avec une charge minérale ou organique, de lactame(s) et/ou de lactone(s) en solution et/ou en suspension dans un liquide organique. On pourra se référer par exemple aux procédés décrits dans les documents EP0192515 et FR2910900. Avantageusement, lesdites particules poreuses dans la présente invention sont sphéroïdales. Elles sont préférentiellement choisies parmi les poudres commercialisées sous la marque Orgasol®.
Selon l'invention, l'agent cosmétique ou pharmaceutique est un agent améliorant l'état des matières kératiniques de l'être humain, comme la peau humaine, les cheveux, les cils ou les ongles, ou encore les lèvres du visage. De préférence, ledit au moins un agent cosmétique ou pharmaceutique est liquide à la température à laquelle est effectué l'ajout dans le procédé selon l'invention. L'ajout peut être fait à toute température, dès l'instant que l'agent cosmétique ou pharmaceutique est liquide au moment de l'ajout. La température d'ajout est judicieusement choisie par l'homme du métier de sorte que l'agent cosmétique est à la fois liquide et stable (ne se dégrade pas à la température d'ajout). De manière avantageuse, l'ajout est fait à température ambiante, par exemple à une température comprise dans la gamme de 15 à 25°C, en utilisant un agent cosmétique ou pharmaceutique liquide et stable à cette température. En effet, l'agent cosmétique ou pharmaceutique est introduit à l'état liquide, c'est-à-dire sous forme d'un liquide pur. En plus de l'agent cosmétique ou pharmaceutique, les particules de poudre obtenues selon le procédé de l'invention peuvent donc renfermer de l'eau, par exemple purifiée ou d'origine thermale. Selon ce mode de réalisation particulier de l'invention, lesdites particules contiennent de l'eau.

Ledit au moins un agent cosmétique ou pharmaceutique utilisé dans la présente invention est choisi parmi les agents hydratants, astringents, antiseptiques, anti-inflammatoires, antimicrobiens, anticellulitiques, antiséborrhéiques, matifiants, absorbants, antipelliculaires, antirides, antioxydants, anti-radicaux libres, lissants, lubrifiants, adoucissants, colorants, anti-solaires, anti-irritants, anti-couperose, cicatrisants, décongestionnants, régénérants, déodorants, parfumants, antitranspirants, anti-perspirants, dépilatoires, stimulateurs de la pousse du cheveu ou du cil, dépigmentants, filmogènes, adhérants, fixants, pigmentants, nacrants, auto-bronzants, photodynamiseurs de pigmentation, vitamines, et leurs mélanges.

Les agents cosmétiques ou pharmaceutiques utilisés dans la présente invention sont ceux classiquement utilisés dans les domaines cosmétique et dermatologique. Ils sont généralement à base de molécules organiques ou inorganiques. Les agents cosmétiques ou pharmaceutiques à base de molécules organiques conviennent particulièrement bien pour la présente invention.

A titre d'exemples d'agents cosmétiques ou pharmaceutiques, on peut citer notamment :
- les agents hydratants et nourrissants, tels que les acides aminés (glycine, alanine, thréonine, citrulline), les composants de la sueur (acide lactique, chlorure de sodium, l'urée, la serine); les alpha-hydroxyacides (AHA), les sucres (le mannose, le fructose, le galactose, la N-acetyl glucosamine); et les produits insaponifiables issus des huiles naturelles telles que l'huile de jojoba, d'olive ou de soja; les céramides naturelles ou synthétiques (tels que oleoyldihydrosphingosine) ;
- les astringents, tels que les sels d'aluminium, et les agents réducteurs des pores tels que ceux décrits dans la demande de brevet WO 02/32392 ;
- les antiseptiques tels que le trichlorocabanilide (TCC), dichlorophène, bromochlorophène, triclosan, ou encore les huiles essentielles comme le thym, le romarin, la sarriette, l'origan ;
- les anti-inflammatoires, les anti-irritants, tels que l'alpha-bisabolol, le glycyrrhizinate de dipotasium, l'acide glycyrrhétinique et ses dérivés, l'acide ellagique, l'acide ursolique, l'ibuprofène, le naproxen, le fénoprofen, le carprofen, le kétoprofène, les anti-inflammatoires stéroïdiens tels que la cortisone, la pregnénolone, le désonide et les melanges d'alcanolamines et de tyrosine tels que ceux décrits dans la demande de brevet EP 1192 939, les extraits de romarin ;
- les antimicrobiens, bactériostatiques, bactéricides, fongistatiques, fongicides ;
- les anticellulitiques, tels que la caféine, l'escine, la lécithine, la forskoline, la carnitine, la tétrahydroxypropyl ethylènediamine, l'extrait naturel des graines de Chenopodium quinoa ;
- les antiséborrhéiques, tels que les dérivés d'iminodibenzyle ou de fluorène tels que décrits dans le brevet US6355687, les dérivés d'amines secondaires substituées tels que décrits dans le brevet US6355686, les dérivés d'acide glucuronique et de glucosamine et leurs sels, tels que décrits dans la demande de brevet EP1219296, les associations de niacinamides avec un alkyle ou un ester d'alcényle en C11-C30 d'acide salicylique tels que décrits dans la demande de brevet WO02/067 889 ;
- les agents kératolytiques ou prodesquamants, par exemple les alpha-hydroxy-acides, les beta-hydroxy-acides, les alpha-céto-acides, les béta-céto-acides, les retinoïdes et leurs esters, le rétinal, l'acide retinoïque et ses dérivés ;
- les matifiants, les absorbants, tels que l'argile, le kaolin, des antilipases, du lactate d'éthyle ;
- les antipelliculaires, tels que la pyrithione de zinc ;
- les antirides, les agents lissants, tels que les protéines ou leurs hydrolisats, le collagène, l'élastane, les huiles végétales, les acides gras polyinsaturés, les raffermissants,
- les antioxydants, tels que le butylhydroxytoluène (BHT), le butylhydroxyanisole (BHA), le propyl gallate, l'octyl gallate, les caroténoïdes tels que le beta-carotène, le lycopène, la canthaxanthine, l'ubiquinone, le tetra hydroxycinnamate de dibutyl pentaerythrityle, la vitamine E, le trolox, la vitamine C et ses dérivés ;
- les anti-radicaux libres, tels que la vitamine E, la caféine, le mannitol, les extraits de mélisse et certaines enzymes telles que les superoxydes dismutases, la glutathion peroxydase, les catalases ;
- les adoucissants, les lubrifiants, tels que les silicones ;
- les colorants, d'origine minérale, animale (carmin), végétale (naphtoquinones, anthraquinones), ou synthétique ;
- les anti-solaires, les filtres UVA et/ou UVB, les pigments ou les nanopigments, on peut citer comme filtres: l'acide p-aminobenzoique et ses dérivés (esters de glycéryle, d'éthyle, d'isobutyle); les anthranilates (comme l'oaminobenzoate ou ses esters d'alkyle); les salicylates (esters d'amyle, phényle, benzyle, dipropylène glycol); les dérivés de l'acide cinnamique (esters d'amyle, de benzyle); les dérivés de l'acide dihydroxycinnamique (umbelliférone); les dérivés de l'acide trihydroxycinnamique (esculitine); des hydrocarbures (stilbène); les dibenzalacétone et benzalacétophénone; les dérivés de coumarine; les benzophénones hydroxy- ou methoxy- substituées; l'acide tannique et ses dérives; les benzophénones et tout autre filtre antisolaire classiquement utilise dans le domaine cosmétique et/ou dermatologique comme le benzylidene camphre, l'acide benzene 1,4 [di(3-methylidene campho-10-sulfonique)], ou encore le méthylsulfate de 4-(3-méthylidène camphre) phényl trimethyl-ammonium, le 2 cyano-3,3'-diphénylacrylate de 2-éthylhexyle ou encore le dibenzoylméthane ainsi que leurs mélanges ; comme pigments ou nanopigments, on peut citer les oxydes de zinc et/ou de titane (TiO2, ZnO2) ;
- les anti-couperose, actifs sur la microcirculation, vasoconstricteurs, tels que l'hamamélis, les extraits fluides de mélilot, d'aubépine, de ruscus, du sulfate de dextran, de ratanhia, de marron d'inde ;
- les cicatrisants, tels que le sucralfate, le Centella asiatica ;
- les décongestionnants, tels que l'eaude bleuet, azulène, alpha-bisabolol ;
- les régénérants, tels que la prêle ;
- les déodorants, tels que le ricinoléate de zinc, les dérivés des acides lactique ou tartrique, des antiseptiques tels que ceux déjà décrits ;
- les agents parfumants et matières aromatiques d'origine naturelle végétale ou animale, d'origine synthétique.
- les antitranspirants, les anti-perspirants, tels que les sels d'aluminium et de zirconium, l'alun de potassium, les extraits de feuilles de buchu, hamamélis ;
- les dépilatoires, tels que les dérivés organiques soufrés, les sels d'acide thioglycoliues et thiolactiques ;
- les agents inhibiteurs de la pousse du cheveu ou du poil tels que les serines protéases décrites dans le brevet US 6 407 056, l'acide caféique, la quercétine, le gallate de propyle, l'acide norhydroguaiarétique ou NDGA, l'indométhacine, l'hydrochlorure d'eflornithine, les extraits végétaux tels décrits dans le brevet US 6 171 595 comme les extraits de clou de girofle, de bouton de rose, de pimprenelle (burnet), de gambir..., les composes décrits dans le brevet US 6 075 052, le tétramisole, l'orthovanadate de sodium, le levamisole, le chromoglycate disodique, le nitrate de vanadium et le nitrate de gallium tel que décrit dans le brevet US 6 020 006, ainsi que les composes décrits dans les brevets US 4 885 289, US 4 720 489, US 5 132 293, US 5 096 911, US 5 095 007, US 5 143 925, US 5 328 686, US 5 440 090, US 5 364 885, US 5 411 991, US 5 648 394, US 5 468 476, US 5 475 763, US 5 455 608, US 5 674 477, US 5 728 736 et 5 652 273 et dans les demandes de brevet WO 94/27586, WO 94/27563 et WO 98/03149. On pourra également utiliser les extraits de genévrier tels que decrits dans le brevet US 6 375 948 ;
- les agents anti-pelliculaires tels que la pyrithione de zinc ;
- les agents inhibiteurs de la chute du cheveu ainsi que les stimulateurs de la pousse du cheveu ou du cil, tels que le minoxidil, la biotine, le finastèride, le 2,4 dipirymidine N-oxyde, le panthénol et leurs dérivés, la flavanone T, ou plus généralement tout extrait végétal, possédant une activité anti 5 alpha reductase de type I ou II,
- les agents dépigmentants, tels que l'hydroquinone, l'acide ascorbique et ses dérivés, l'ascorbylphosphate de magnésium, l'acide kojique
- les agents filmogènes, adhérants, fixants,
- les pigmentants, les pigments minéraux, les pigments de synthèse ;
- les nacrants, d'origine naturelle organique, minérale, ou synthétique
- les auto-bronzants, tels que la dihyfroxyacétone ou DHA,
- les photodynamiseurs de pigmentation, tels que les dérivés de la tyrosine, les précurseurs mélaniques, tels que l'indol-5,6-quinone ;
- les vitamines, telles que la vitamine A, B, B2, B3, B5, B6, B8, la vitamine C, K, la vitamine F, H, la vitamine PP, la vitamine D, D2, D3, et leurs dérivés ;
- et leurs mélanges.

Selon un mode de réalisation préféré de la présente invention, ledit au moins un agent cosmétique ou pharmaceutique est choisi parmi les agents hydratants, c'est-à-dire les émollients, humectants, relipidants, et leurs mélanges. En effet, parmi les hydratants, on peut citer les émollients qui, occlusifs, retiennent l'eau sur la peau humaine, les lèvres du visage ou les phanères, comme les céramides, les huiles essentielles, etc ; et les humectants qui captent l'eau comme les polyols tels que la glycérine, le propylène glycol, le sorbitol, etc. De préférence, ledit au moins un agent cosmétique ou pharmaceutique est choisi parmi les polyols tels que la glycérine, le propylène glycol ; les glycérides, tels que les triglycérides ; le hyaluronate de sodium ; l'acide lactique ; les lipides ; les céramides, tels que le céramide-3 ; et leurs mélanges.

Avantageusement, ledit au moins un agent cosmétique ou pharmaceutique comprend un composé de formule chimique identique à celle d'un composé cutané, c'est-à-dire présent naturellement dans une peau humaine saine. Avantageusement, ledit au moins un agent cosmétique ou pharmaceutique est issu de la biofermentation. Ce procédé d'obtention repose sur la fermentation naturelle, un procédé de biotechnologie consistant à laisser fermenter des levures qui vont produire spontanément l'agent cosmétique ou pharmaceutique, par exemple l'acide hyaluronique. Ainsi, l'agent cosmétique ou pharmaceutique obtenu présente le taux le plus haut de pureté disponible, puisqu'il est sans aucun résidu de solvant ou d'impureté. De plus, ces procédés biotechnologiques permettent de maîtriser la distribution des masses moléculaires de l'agent cosmétique ou pharmaceutique. Par exemple l'utilisation de hyaluronate de sodium issu de la biotechnologie de poids moléculaire strictement inférieur à 10⁶ Daltons, de préférence inférieur à 350000 Daltons, de préférence inférieur à 250000 Daltons permet d'augmenter le taux de hyaluronate de sodium que l'on peut incorporer dans les particules poreuses. Cette gamme de poids moléculaire maîtrisé permet aussi une meilleure pénétration de l'agent cosmétique, ici du hyaluronate de sodium, dans les couches supérieures de l'épiderme, comme l'indiquent des mesures de cornéométrie, de perte insensible en eau, et de cohésion de la peau. En particulier, l'imprégnation selon le procédé de l'invention de particules poreuses imprégnées d'un tel hyaluronate de sodium dissout dans l'eau permet d'introduire de l'eau encapsulée dans lesdites particules poreuses dans des formulations anhydres, telles que les rouges à lèvres, les poudres compactes, les fonds de teint coulés.

La présente invention a également pour objet des particules de poudre libre à base de polyamide susceptibles d'être obtenues selon le procédé précédemment décrit, lesdites particules étant poreuses, de SSA supérieure à 8m²/g et ayant une teneur d'au moins 25% en poids d'au moins un agent cosmétique ou pharmaceutique autre que l'eau, ledit agent cosmétique ou pharmaceutique étant, à une température comprise dans la gamme de 15 à 25°C, sous la forme d'un liquide pur. Avantageusement, ledit au moins un agent cosmétique ou pharmaceutique représente au moins 40% en poids des particules de poudre.

De préférence, lesdites particules ont un diamètre moyen en volume compris dans la gamme allant de 3 à 12 µm, de préférence de 5 à 10 µm. De préférence, lesdites particules à base de polyamide sont choisies parmi les particules de polyamide, de copolyamide, de copolyesteramide, et leurs mélanges. Avantageusement, lesdites particules à base de polyamide comprennent une teneur en pourcentage molaire de polyamide 12 d'au moins 50%. Selon un mode de réalisation préféré de la présente invention, lesdites particules de poudre à base de polyamide sont obtenues au moins en partie par polymérisation anionique, par ensemencement avec une charge minérale ou organique, de lactame(s) et/ou de lactone(s) en solution et/ou en suspension dans un liquide organique. De manière encore plus préférée, lesdites particules sont sphéroïdales.

L'intérêt d'utiliser des poudres poreuses contenant majoritairement du polyamide 12 comme support d'agents cosmétiques ou pharmaceutiques est leur très grande compatibilité avec les constituants de la surface de la peau. La structure moléculaire des chaînes de polyamides 12 est en plusieurs points similaire à celle des céramides, constituants principaux des couches supérieures de l'épiderme. Les chaînes de polyamide 12 contiennent des longs segments (motifs) hydrocarbonés à 12 atomes de carbone, formant des chaînes grasses lipophiles, similaires à celles des constituants lipidiques de la peau. Elles assurent une grande compatibilité avec la peau : douceur, non irritant, excellente rémanence et une certaine pénétration dans la couche cornée facilitée par une granulométrie de 5 à 10 µm. Enfin, les fonctions amides des chaînes de Polyamide 12 sont des fonctions polaires qui établissent des liaisons hydrogènes avec les fonctions amides des céramides de la peau, renforcent la compatibilité entre les particules de l'invention et la surface de la peau et prolongent l'adhésion des particules à la surface de la peau. Ceci permet de prolonger le contact entre les poudres chargées d'agent cosmétique ou pharmaceutique et la surface de la peau et optimise l'efficacité de ces agents à la surface de la peau.

En particulier, les poudres microporeuses qui contiennent majoritairement du polyamide 12 (teneur molaire en polyamide 12 supérieure ou égale à 50%) et ayant une granulométrie moyenne inférieure ou égale à 12 µm possèdent des propriétés de compatibilité optimale avec les couches supérieures de la couche cornée.

Les particules de poudre selon l'invention constituent des supports d'agents cosmétiques très stables qui peuvent généralement contenir jusqu'à 50% en poids d'agents cosmétiques ou pharmaceutiques sur le poids total de poudre imprégnée. Elles sont très stables en température (point de fusion supérieur à 130°C) et permettent d'utiliser les actifs dans des procédés qui dépassent 90°C, tels que les fonds de teint coulés ou les rouges à lèvre.

Avantageusement, ledit au moins un agent cosmétique ou pharmaceutique est, tel que décrit précédemment, choisi parmi les agents hydratants, astringents, antiseptiques, anti-inflammatoires, antimicrobiens, anticellulitiques, antiséborrhéiques, matifiants, absorbants, antipelliculaires, antirides, antioxydants, anti-radicaux libres, lissants, lubrifiants, adoucissants, colorants, anti-solaires, anti-irritants, anti-couperose, cicatrisants, décongestionnants, régénérants, déodorants, parfumants, antitranspirants, anti-perspirants, dépilatoires, stimulateurs de la pousse du cheveu ou du cil, dépigmentants, filmogènes, adhérants, fixants, pigmentants, nacrants, auto-bronzants, photodynamiseurs de pigmentation, vitamines, et leurs mélanges.

De préférence, ledit au moins un agent cosmétique ou pharmaceutique est choisi parmi les agents hydratants, c'est-à-dire choisi parmi les émollients, humectants, relipidants, et leurs mélanges. De préférence, ledit au moins un agent cosmétique ou pharmaceutique est choisi parmi les polyols tels que la glycérine, le propylène glycol ; les glycérides, tels que les triglycérides ; le hyaluronate de sodium ; l'acide lactique ; les lipides ; les céramides, tels que le céramide-3 ; et leurs mélanges.

Selon un mode de réalisation avantageux de la présente invention, ledit au moins un agent cosmétique ou pharmaceutique comprend un composé de formule chimique identique à celle d'un composé cutané, c'est-à-dire présent naturellement dans une peau humaine saine. Par exemple, les céramides sont une famille lipidique de grande importance biologique. La couche externe de la peau (stratum corneum) assure la protection primaire de la peau. Elle se compose à 65% de céramides (lipides). Les céramides extracellulaires permettent la cohésion du stratum corneum et la formation de la barrière épidermique, et participent également au processus de desquamation. En particulier, le céramide 3 soutient la fonction des principaux lipides de céramide et remplit la fonction de barrière efficace contre la déshydratation de la peau. Avec l'âge cependant, la propre production de céramides du corps régresse constamment. La peau perd progressivement sa capacité à lier et stocker l'eau. Elle perd ainsi sa souplesse, son élasticité et sa tonicité. Ainsi, selon un mode de réalisation particulier de l'invention, des particules de poudre à haute teneur en ceramide-3 peuvent venir pallier ce déficit en céramides de la peau. Selon un autre exemple, l'acide hyaluronique participe largement à la régulation de la teneur en eau de la peau et il est, dès lors, indispensable au maintien d'une peau jeune. Ce composant naturel du tissu conjonctif est aujourd'hui synthétisé et peut donc être incorporé dans les particules selon l'invention pour former sur la peau un film aqueux hydratant. Selon encore un autre exemple, l'acide lactique et les lactates sont des composants naturels de l'être humain, aux fonctions multiples : hydratant, humectant, anti-microbien, régulateur de pH, éclaircissant, la forme optiquement active L+ de l'acide lactique étant particulièrement efficace.
De préférence, ledit au moins un agent cosmétique ou pharmaceutique est issu de la biofermentation. Ce peut être le cas par exemple pour l'acide lactique, L(+) acide lactique, l'acide hyaluronique, le céramide-3, qui peuvent être synthétisés par biofermentation, avec tous les avantages précités en terme de pureté, de contrôle des poids moléculaires, etc...

La présente invention a également pour objet l'utilisation de particules de poudre telles que définies précédemment dans les produits cosmétiques, pharmaceutiques ou de parfumerie. Ladite poudre peut notamment être utilisée pour la fabrication d'un produit comprenant un agent cosmétique ou pharmaceutique ayant une diffusion améliorée, c'est-à-dire augmentée et/ou prolongée dans le stratum corneum après application topique dudit produit sur la peau.

La présente invention a également pour objet une composition cosmétique, pharmaceutique, ou de parfumerie caractérisée en ce qu'elle comprend des particules telles que définies précédemment. Ladite composition peut notamment être un produit coloré, non coloré ou transparent choisi parmi les produits suivants :
- produits de maquillage pour le visage et le corps humain, tels que fond de teint, crème teintée, poudre libre ou compacte, fard à paupières, mascara, eye liner, rouge à lèvre ;
- produits de soins pour le visage et le corps humain, tels que crème, lait, lotion, masque, produit de gommage, produits nettoyants et/ou démaquillants, déodorants, antitranspirants, antiperspirants, produits de rasage, produits d'épilation ;
- produits capillaires, tels que shampooings, produits pour la mise en forme des cheveux, produits de maintien de la coiffure, produits antipelliculaires, produits antichute, produits contre la sécheresse des cheveux, teintures capillaires, produits de décoloration ;
- produits de parfumerie, tels que parfum, lait, crème, poudre libre ou compacte parfumée.

### Exemples

Les exemples ci-dessous illustrent la présente invention sans en limiter la portée. Dans les exemples, sauf indication contraire, tous les pourcentages et parties sont exprimés en masse.

### Exemple 1 : procédé d'imprégnation selon l'invention

### Dispositif :

On utilise un réacteur mélangeur équipé : d'un agitateur-racleur de parois, d'un disperseur-émotteur, d'un système de régulation de température, d'un système permettant de travailler sous vide (pompe à vide, piège à azote), d'un balayage de gaz inerte tel que l'azote (facultatif en fonction des agents cosmétiques ou pharmaceutiques)

### Préparation de la poudre :

- Introduire la poudre dans le réacteur ;
- Faire le vide pour dégazer la poudre (30 mm de Mercure) ;
- Mettre la poudre sous agitation (60 tr/min) et maintenir le vide pendant 30 minutes ;
- Isoler le réacteur.

### Préparation de la matière active :

Si l'agent cosmétique ou pharmaceutique est liquide, peu visqueux et suffisamment mouillant, ils peut être imprégné directement sur la poudre.

### Imprégnation de la matière active :

- Introduire l' actif goutte à goutte dans le réacteur, la poudre restant sous agitation.
- Le taux d'imprégnation optimum est atteint lorsque les particules de poudre restent libres et conservant leur coulabilité initiale, c'est-à-dire gardent la même granulométrie qu'avant imprégnation. Elles ne doivent pas commencer à s'agglomérer.
- Pour les actifs fragiles, sensibles à l'oxydation, l'introduction est réalisée sous balayage d'azote.
- Une fois l'introduction terminée, la poudre est maintenue sous agitation pendant 30 minutes.

Pour éviter le mottage de la poudre, il est possible d'utiliser un disperseur ou d'autres systèmes d'agitation rapide équivalents.

On obtient des particules de poudre libre hautement chargées en agent(s) cosmétique(s) ou pharmaceutique(s).

### Exemple 2 : Particules de poudre libre à haute teneur en agent(s) cosmétique(s) ou pharmaceutique(s):

**Tableau 1**

| | **Exemple 2.1** | **Exemple 2.2** |
|---|---|---|
| Agent cosmétique ou pharmaceutique | Céramide-3 | Acide lactique |
| Particules de poudre | Copolyamide PA 6/12 (20%/80% molaire)- 10 µm SSA > 8 m²/g | Polyamide PA12 5 µm SSA > 8 m²/g |
| Solubilisant | Olive glycerides | Eau |
| Séchage | non | non |
| Teneur en agent(s) cosmétique(s) dans les particules de poudre libre | 25% (Céramide-3 + Olive glycerides) | 40% acide lactique |
| Fonction de l'agent | Anti-âge, restaure la couche cornée | Exfoliant, régulateur peau grasse |

Les particules de poudre de PA6/12 et de PA12 utilisées respectivement pour la fabrication des particules de poudre libre imprégnées des exemples 2.1 et 2.2 sont des particules sphéroïdales de la marque Orgasol®, le diamètre indiqué en µm est le diamètre moyen en volume. Celles de PA6/12 sont obtenues selon le procédé décrit dans le document WO 2008/087335, celles de PA12 selon le procédé décrit dans le document EP0192515.

Les particules de poudre libre imprégnées des exemples 2.1 et 2.2 selon l'invention ont respectivement la même granulométrie et la même surface spécifique que les particules de poudre de respectivement PA 6/12 et PA12 non imprégnées en agent cosmétique.

Dans l'exemple 2.1, le solubilisant du céramide-3, est issu de mélanges de tri-glycérides, di-glycérides et mono-glycérides, obtenus par estérification d'acides gras d'origine végétale (huile d'olive en particulier). Ce solubilisant est un composé lipophile présentant une grande affinité pour la peau, et qui présente un toucher léger et non gras. En plus de ses qualités cosmétiques, ce solubilisant est utilisé pour solubiliser des agents cosmétiques particulièrement difficile à solubiliser, tels que les céramides, notamment le céramide-3. Le procédé permet donc d'introduire dans des particules poreuses de poudre libre des céramides qui seront ensuite réellement disponibles pour les couches supérieures de l'épiderme. L'efficacité de ces particules est démontrée (voir tests d'efficacité dans l'exemple 4.1) dans une formulation de composition de fond de teint (voir exemple 3.1).

Dans l'exemple 2.2, l'acide lactique est issu de la biofermentation. Dans ces particules de poudre libre, l'association d'acide lactique issu de biotechnologie et de particules poreuses contenant majoritairement du polyamide 12 permet d'augmenter l'efficacité du produit cosmétique qui les contient, notamment grâce à la haute teneur des particules en acide lactique (40% au lieu de 20% dans les particules imprégnées de l'art antérieur). Des tests d'efficacité de ces particules montrent qu'il y a une synergie entre les poudres poreuses contenant majoritairement du polyamide 12 et l'acide lactique sur le contrôle du sébum à la surface de la peau. L'efficacité de ces particules est démontrée (voir tests d'efficacité dans l'exemple 4.2) dans une formulation de composition de lait démaquillant (voir exemple 3.2).

Enfin, les particules (Exemples 2.1 et 2.2) ne contiennent aucune trace de solvant nocif pour la peau.

La mesure de la granulométrie des poudres dans la présente description, notamment dans les exemples et comparatifs, est réalisée à l'aide d'un granulomètre de marque Coulter LS230. Il permet d'obtenir la distribution granulométrique des poudres de laquelle on peut déterminer le diamètre moyen et la largeur de la distribution ou l'écart-type de la distribution. La distribution granulométrique des poudres selon l'invention est déterminée selon les techniques habituelles à l'aide d'un granulomètre Coulter LS230 de la société Beckman-Coulter. A partir de la distribution granulométrique, il est possible de déterminer le diamètre moyen en volume avec le mode de calcul logarithmique version 2.11a. du logiciel, ainsi que l'écart-type qui mesure le resserrement de la distribution ou la largeur de la distribution autour du diamètre moyen.

### Exemple 3: Compositions comprenant des particules imprégnées d'un agent cosmétique:

### - Exemple 3.1: Fabrication d'un fond de teint coulé selon la formulation suivante :

**Tableau 2**

| **Phase** | **INCI** | **%** |
|---|---|---|
| **A** | ARACHIDYL ALCOHOL,BEHENYL ALCOHOL,ARACHIDYL GLUCOSIDE | 2,00 |
| | CETYLPALMITATE | 0,50 |
| | CETEARYL ALCOHOL | 0,30 |
| | C18-21 ALKANE | 4,00 |
| | C13-15 ALKANE | 2,00 |
| | NEOPENTYL GLYCOL DIHEPTANOATE | 3,00 |
| | DIMETHICONE | 1,50 |
| | POLYGLYCERYL -3 DIISOSTEARATE | 1,00 |
| | ETHYLHEXYLMETHOXYCINNAMATE | 4,00 |
| | BUTYLMETHOXYDIBENZOYL METHANE | 0,50 |
| **B** | AQUA | qsp 100 |
| | EDTA | 0,1 |
| | GLYCERIN | 2 |
| | METHYLPROPANEDIOL | 4 |
| | MAGNESIUM ALUMINUM SILICARE | 0,4 |
| | CETEARYL SULPHATE | 0,2 |
| | XANTHAN GUM | 0,15 |
| **C** | MICA | 1,5 |
| | CI77499 | 0,1 |
| | CI77491/2/9 | 0,4 |
| | CI77492 | 1,5 |
| | CI77891 | 6 |
| **D** | **Exemple 2.1** | 3,00 |

Peser les ingrédients de la phase A, les charger dans un fondoir et chauffer à 110°-115°, en mélangeant à vitesse constante. Vérifier l'homogénéité de la phase puis refroidir à 85-90°C. Ajouter alors un à un, les ingrédients de la phase B en mélangeant continuellement. Avant de poursuivre, vérifier l'homogénéité du mélange. Pendant ce temps, mélanger les poudres de la phase C. Puis ajouter la phase C dans le fondoir en mélangeant à vitesse constante jusqu'à ce que l'ensemble soit homogène. Maintenir la température à 85-90°C. Ajouter un à un les ingrédients de la phase D, et mélanger à vitesse constante jusqu'à ce que l'ensemble soit homogène. Couler dans les moules métalliques appropriés en maintenant le mélange à 85°C. Après refroidissement, on obtient un fond de teint coulé.

### - Exemple 3.2 : Composition d'un lait démaquillant avec D, et avec D1 ou D2 :

**Tableau 3**

| **Phase** | **INCI** | % |
|---|---|---|
| **A** | AQUA | qsp 100 |
| | DISODIUM EDTA | 0,15 |
| | HYDROPYPROPYL GUAR | 0,05 |
| | XANTHAN GUM | 0,15 |
| | GLYCERIN | 3,00 |
| **B** | PEG 8 stearate - cetearyl ethylhexanoate - isopropyl myristate - glyceryl stearate - stearyl heptanoate - cetyl alcohol - butyl stearate - cetyl palmitate - sorbitan sesquioleate - stearic acid - aqua - potassium hydroxyde - BHA | 8,00 |
| | CETEARYL ALCOHOL | 1,50 |
| | BUTYROSPERMUM PARKII BUTTER | 2,00 |
| | ISONONYL ISONONANOATE | 4,00 |
| | MACADAMIA TERNIFOLIA SEED OIL | 8,00 |
| | BETASITOSTEROL | 0,20 |
| | phenoxyethanol - methylparaben ethylparaben - butylparaben propylparaben - isobutylparaben | 0,80 |
| **C** | AQUA | 5,00 |
| | IMIDAZOLIDYNYL UREA | 0,30 |
| | SODIUM COCOAMPHOACETATE | 2,00 |
| **D** | | |
| **Pour Exemple 3.2** | **Particules de poudre libre de l'Exemple 2.2** | 2,00 |
| | Nylon 11 (RILSAN T NAT BHV COS) | 2,00 |
| **ou D1** | | |
| Pour Comparatif 1 | Polyamide PA12 (5 µm, SSA> 8m²/g) (Orgasol ®) | 1,20 |
| | LACTIC ACID | 0,80 |
| | Nylon 11 | 2,00 |
| **ou D2** | | |
| Pour Comparatif 2 | | |
| | Nylon 11 | 2,00 |

Mélanger tous les composants de la phase A. Ajouter la phase B sous agitation, puis les phases C et D (ou D1 ou D2) successivement. Contrôler le pH du lait démaquillant obtenu.

### Exemple 4 : Tests d'efficacité sur une composition selon l'invention

Les compositions de l'exemple 3 sont testées sur des panels d'experts entraînés.

### - Exemple 4.1 :

La composition de l'exemple 3.1 (fond de teint coulé) est comparée à une composition placebo (comparatif 3) dans laquelle les particules de poudre libre ont été remplacées par la même teneur en talc (3%).

On compare la perte insensible en eau (aussi appelé TEWL, «transepidermal water loss») obtenue après utilisation de ces deux compositions. L'eau trans-épidermique est celle qui se diffuse à travers la peau avant de remonter à sa surface. Elle s'en évapore alors, et est perdue. Plus la couche cornée est abîmée, plus la perte insensible en eau augmente.

On utilise la méthode bien connue du Tewameter®, au moyen d'un appareil Tewameter® 300 (Courage + Khazaka, electronic GmbH).

**Tableau 4**

| Mesure de la variation (%) de TEWL «transepidermal water loss» | Après 7 jours d'utilisation | Après 15 jours d'utilisation | Après 30 jours d'utilisation | Après 60 jours d'utilisation |
|---|---|---|---|---|
| Comparatif 3 | -1% | -2% | -3% | -3% |
| **Exemple 3.1** | -6% | -8% | -10% | -12% |

L'application quotidienne sur le visage de la composition de l'exemple 3.1 entraîne une amélioration de la fonction barrière de la peau, qui se caractérise par une diminution de la perte insensible en eau TEWL de 12% après 60 jours d'utilisation du fond de teint de l'exemple 3 .1. La diminution de TEWL n'est que de 3% pour la même composition de fond de teint placebo ne comprenant pas de particules (comparatif 3).

On compare le niveau de sébum obtenu après utilisation de ces deux compositions (comparatif 3 et exemple 3.1), par la méthode Sebumeter® à l'aide d'un sébumètre de marque Sebumeter® 815, Courage + Khazaka GmbH.

**Tableau 5**

| Mesure de la variation (%) du niveau de sébum | Après 7 jours d'utilisation | Après 15 jours d'utilisation | Après 30 jours d'utilisation | Après 60 jours d'utilisation |
|---|---|---|---|---|
| Comparatif 3 | +2% | +2% | +3% | +3% |
| **Exemple 3.1** | +4% | +8% | +14% | +18% |

L'utilisation quotidienne de la composition de l'exemple 3.1 améliore de manière continue le film hydrolipidique de la peau, comme le montre l'évolution du niveau de sébum. Pour le comparatif 3, le niveau de sébum reste inchangé.

On compare les variations du paramètre « aspect lisse de la peau » obtenu après utilisation de ces deux compositions (comparatif 3 et exemple 3.1), par la méthode de l'évaluation de l'état de surface de la peau « Skin surface évaluation », au moyen d'un appareil visioscan VC 98 (Courage + Khazaka electronic GmbH) utilisé pour l'analyse in vivo et en 3D de la surface de la peau. Le paramètre mesuré par l'analyse d'image est le SESm (skin smoothness) qui est calculé à partir de la largeur moyenne de ride et la profondeur moyenne de ride. Plus la valeur de SESm est faible et plus la peau a un aspect lisse.

**Tableau 6**

| Mesure de la variation (%) du paramètre «aspect lisse de la peau» | Après 7 jours d'utilisation | Après 15 jours d'utilisation | Après 30 jours d'utilisation | Après 60 jours d'utilisation |
|---|---|---|---|---|
| Comparatif 3 | +3% | +5% | +6% | +6% |
| **Exemple 3.1** | +8% | +11% | +12% | +14% |

L'utilisation du fond de teint de l'exemple 3.1 augmente l'aspect lisse de la peau de 14% après 60 jours d'utilisation quotidienne. Selon la nature de la peau, l'augmentation du lissage de la peau peut atteindre 30% avec la composition de l'exemple 3.1. La formule placebo (comparatif 3) n'a amélioré l'aspect lisse de la peau que de 6%, plateau atteint au bout de 30 jours.

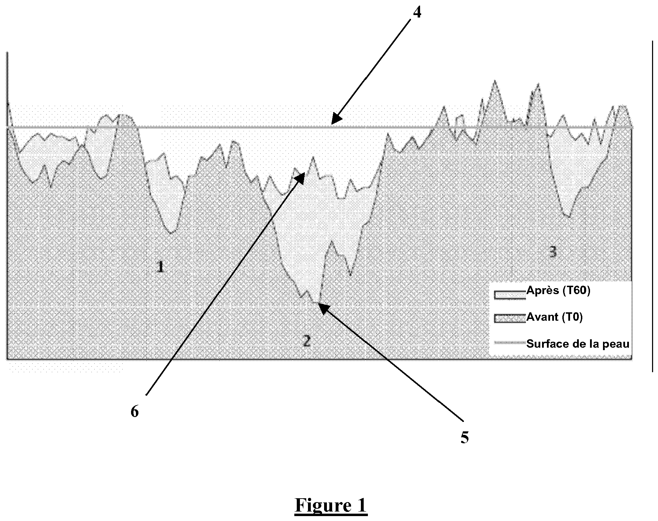

Les particules imprégnées (Exemple 2.1) ont une efficacité à long terme sur la réduction de l'aspect rugueux de la peau et sur la réduction de la profondeur des rides. La figure 1 est un graphe représentant une section de peau à travers trois rides référencées 1, 2, et 3 (en abscisse) de différentes profondeurs (en ordonnée). Le niveau (profondeur) des rides est représenté avant (en gris foncé, 5) et après (en gris clair, 6) 60 jours d'utilisation du fond de teint de l'exemple 3.1. Sur ce graphe, on remarque que la profondeur (6) des rides est réduite de manière significative après 60 jours d'utilisation par rapport à la profondeur (5) des rides avant utilisation de la composition, pour se rapprocher de la surface (4) de la peau (représentée par une ligne horizontale sur la partie haute du graphe).

### - Exemple 4.2:

La composition de l'exemple 3.2 (lait démaquillant) est comparée à deux compositions :
- une composition appelée comparatif 1, dans laquelle les particules de poudre libre chargées en agent cosmétique (acide lactique) (de l'exemple 3.2) ont été remplacées par des teneurs équivalentes d'acide lactique et de poudre libre à base de polyamide de même type mais non imprégnée par l'acide lactique.
- une composition appelée comparatif 2, qui ne contient pas de particules de poudre libre chargées en agent cosmétique.

On mesure la quantité de sébum à la surface de la peau avant l'application du lait démaquillant, puis 2 heures après la première application, et enfin après une semaine d'application journalière.

La mesure de la quantité de sébum à la surface de la peau est faite selon la méthode de mesure bien connue du sebumètre (la référence du sebumètre utilisé est : Sebumètre 815, Courage +Khaza GmbH). C'est une mesure directe de l'accumulation du sebum sur la peau.

**Tableau 7**

| Mesure | **Exemple 3.2** | Comparatif 1 | Comparatif 2 |
|---|---|---|---|
| Variation (réduction en %) du niveau de sébum, 2 heures après l'application du lait démaquillant | -30% | -20% | -5% |

L'utilisation du lait démaquillant de l'exemple 3.2 réduit le sébum à la surface de la peau d'au moins 30%. L'utilisation du lait démaquillant du comparatif 1 ne réduit le sébum que de 20%, et celle du comparatif 2 ne réduit le sébum que de 5%.

Cette différence d'efficacité entre les compositions de lait démaquillant de l'exemple 3.2 et du comparatif 1 est maintenue même après 6 mois de stockage. Ceci confirme la stabilité des particules de poudre libre à haute teneur en agent cosmétique ou pharmaceutique dans les formulations les comprenant.

Les particules de poudre libre à haute teneur en agent cosmétique ou pharmaceutique « boostent » ou augmentent l'efficacité de l'agent qu'elles contiennent. De plus, on remarque que l'action observée pour les formulations contenant des particules selon l'exemple 2.2 n'est pas suivie d'un effet rebond, puisque, après le démaquillage le niveau de sébum n'augmente pas de nouveau, mais au contraire reste stable et correspond au niveau recherché pour une peau normale (autour de 190 µg/cm²), également après 7 jours d'utilisation journalière du lait démaquillant de l'exemple 3.2, comme le montre le tableau 8.

**Tableau 8**

| | Avant application du lait démaquillant de l'exemple 3.2 | 2 heures après l'application du lait démaquillant de l'exemple 3.2 | Après 7 jours d'utilisation journalière du lait démaquillant de l'exemple 3.2 |
|---|---|---|---|
| Niveau de sébum (gg/cm²) | 265 | 188 | 185 |
| Classification | peau grasse (>220) | peau normale (100-220) | peau normale (100-220) |

## Revendications

1. Procédé de fabrication de particules de poudre libre à base de polyamide imprégnée d'au moins un agent cosmétique ou pharmaceutique, ledit agent cosmétique étant, à une température comprise dans la gamme de 15 à 25°C, sous la forme d'un liquide pur;
ledit procédé étant constitué des étapes successives suivantes :
- ajout goutte à goutte ou par pulvérisation dudit agent cosmétique ou pharmaceutique à l'état liquide à des particules de poudre libre sous agitation de sorte qu'il représente au moins 25% en poids des particules de poudre, lesdites particules étant poreuses et de SSA supérieure à 8m²/g,
- arrêt de l'ajout avant que les particules de poudre ne commencent à s'agglomérer,
- maintien de l'agitation pendant au moins 5 minutes, puis
- récupération de la poudre libre obtenue imprégnée dudit agent cosmétique ou pharmaceutique.

2. Procédé selon la revendication 1, dans lequel ledit au moins un agent cosmétique ou pharmaceutique représente au moins 40% en poids des particules de poudre.

3. Procédé selon l'une quelconque des revendications 1 ou 2, dans lequel lesdites particules ont un diamètre moyen en volume compris dans la gamme allant de 3 à 12 µm.

4. Procédé selon l'une quelconque des revendications précédentes, dans lequel lesdites particules à base de polyamide sont choisies parmi les particules de polyamide, de copolyamide, de copolyesteramide, et leurs mélanges.

5. Procédé selon l'une quelconque des revendications précédentes, dans lequel lesdites particules à base de polyamide comprennent, avant imprégnation, une teneur en pourcentage molaire de polyamide 12 comprise dans la gamme allant de 50% à 100%, de préférence de 80% à 100%.

6. Procédé selon l'une quelconque des revendications précédentes, dans lequel lesdites particules de poudre à base de polyamide sont obtenues au moins en partie par polymérisation anionique, par ensemencement avec une charge minérale ou organique, de lactame(s) et/ou de lactone(s) en solution et/ou en suspension dans un liquide organique.

7. Procédé selon l'une quelconque des revendications précédentes, dans lequel lesdites particules sont sphéroïdales.

8. Procédé selon l'une quelconque des revendications précédentes, dans lequel lesdites particules contiennent de l'eau.

9. Procédé selon l'une quelconque des revendications précédentes, dans lequel ledit au moins un agent cosmétique ou pharmaceutique est choisi parmi les agents hydratants, astringents, antiseptiques, anti-inflammatoires, antimicrobiens, anticellulitiques, antiséborrhéiques, matifiants, absorbants, antipelliculaires, antirides, antioxydants, anti-radicaux libres, lissants, lubrifiants, adoucissants, colorants, anti-solaires, anti-irritants, anti-couperose, cicatrisants, décongestionnants, régénérants, déodorants, parfumants, antitranspirants, anti-perspirants, dépilatoires, stimulateurs de la pousse du cheveu ou du cil, dépigmentants, filmogènes, adhérants, fixants, pigmentants, nacrants, auto-bronzants, photodynamiseurs de pigmentation, vitamines, et leurs mélanges.

10. Procédé selon la revendication 9, dans lequel ledit au moins un agent cosmétique ou pharmaceutique est choisi parmi les agents émollients, humectants, relipidants, et leurs mélanges.

11. Procédé selon la revendication 9 ou 10, dans lequel ledit au moins un agent cosmétique ou pharmaceutique est choisi parmi les polyols tels que la glycérine, le propylène glycol ; les glycérides, tels que les triglycérides ; le hyaluronate de sodium ; l'acide lactique ; les lipides ; les céramides, tels que le céramide-3 ; et leurs mélanges.

12. Procédé selon l'une quelconque des revendications précédentes, dans lequel ledit au moins un agent cosmétique ou pharmaceutique est issu de la biofermentation.

13. Particules de poudre libre à base de polyamide susceptibles d'être obtenues selon le procédé de l'une quelconque des revendications 1 à 12, lesdites particules étant poreuses, de SSA supérieure à 8m²/g et ayant une teneur d'au moins 25% en poids d'au moins un agent cosmétique ou pharmaceutique autre que l'eau, ledit agent cosmétique étant, à une température comprise dans la gamme de 15 à 25°C, sous la forme d'un liquide pur.

14. Particules selon la revendication 13, dans lesquelles ledit au moins un agent cosmétique ou pharmaceutique représente au moins 40% en poids des particules de poudre.

15. Particules selon l'une quelconque des revendications 13 ou 14, dans lesquelles lesdites particules ont un diamètre moyen en volume compris dans la gamme allant de 3 à 12 µm

16. Particules selon l'une quelconque des revendications 13 à 15, dans lesquelles lesdites particules à base de polyamide sont choisies parmi les particules de polyamide, de copolyamide, de copolyesteramide, et leurs mélanges.

17. Particules selon l'une quelconque des revendications 13 à 16, dans lesquelles lesdites particules à base de polyamide comprennent une teneur en pourcentage molaire de polyamide 12 d'au moins 50%.

18. Particules selon l'une quelconque des revendications 13 à 17, dans lesquelles lesdites particules de poudre à base de polyamide sont obtenues au moins en partie par polymérisation anionique, par ensemencement avec une charge minérale ou organique, de lactame(s) et/ou de lactone(s) en solution et/ou en suspension dans un liquide organique.

19. Particules selon l'une quelconque des revendications 13 à 18, dans lesquelles lesdites particules sont sphéroïdales.

20. Particules selon l'une quelconque des revendications 13 à 19, dans lesquelles ledit au moins un agent cosmétique ou pharmaceutique est choisi parmi les agents hydratants, astringents, antiseptiques, anti-inflammatoires, antimicrobiens, anticellulitiques, antiséborrhéiques, matifiants, absorbants, antipelliculaires, antirides, antioxydants, anti-radicaux libres, lissants, lubrifiants, adoucissants, colorants, anti-solaires, anti-irritants, anti-couperose, cicatrisants, décongestionnants, régénérants, déodorants, parfumants, antitranspirants, anti-perspirants, dépilatoires, stimulateurs de la pousse du cheveu ou du cil, dépigmentants, filmogènes, adhérants, fixants, pigmentants, nacrants, auto-bronzants, photodynamiseurs de pigmentation, vitamines, et leurs mélanges.

21. Particules selon la revendication 20, dans lesquelles ledit au moins un agent cosmétique ou pharmaceutique est choisi parmi les agents émollients, humectants, relipidants, et leurs mélanges.

22. Particules selon la revendication 20 ou 21, dans lesquelles ledit au moins un agent cosmétique ou pharmaceutique est choisi parmi les polyols tels que la glycérine, le propylène glycol ; les glycérides, tels que les triglycérides ; le hyaluronate de sodium ; l'acide lactique ; les lipides ; les céramides, tels que le céramide-3 ; et leurs mélanges.

23. Particules selon l'une quelconque des revendications 13 à 22, dans lesquelles ledit au moins un agent cosmétique ou pharmaceutique est issu de la biofermentation.

24. Utilisation de particules de poudre selon l'une quelconque des revendications 13 à 23 dans les produits cosmétiques, pharmaceutiques ou de parfumerie.

25. Composition cosmétique, pharmaceutique, ou de parfumerie **caractérisée en ce qu'**elle comprend des particules telles que définies dans l'une quelconque des revendications 13 à 23.

26. Composition selon la revendication 25, ladite composition étant un produit coloré, non coloré ou transparent choisi parmi les produits suivants :
- produits de maquillage pour le visage et le corps humain, tels que fond de teint, crème teintée, poudre libre ou compacte, fard à paupières, mascara, eye liner, rouge à lèvre ;
- produits de soins pour le visage et le corps humain, tels que crème, lait, lotion, masque, produit de gommage, produits nettoyants et/ou démaquillants, déodorants, antitranspirants, antiperspirants, produits de rasage, produits d'épilation ;
- produits capillaires, tels que shampooings, produits pour la mise en forme des cheveux, produits de maintien de la coiffure, produits antipelliculaires, produits antichute, produits contre la sécheresse des cheveux, teintures capillaires, produits de décoloration ;
- produits de parfumerie, tels que parfum, lait, crème, poudre libre ou compacte parfumée.

## Patentansprüche

1. Verfahren zur Herstellung von Teilchen von losem Pulver auf Basis von Polyamid, das mit mindestens einem kosmetischen oder pharmazeutischen Mittel imprägniert ist, wobei das kosmetische Mittel bei einer Temperatur im Bereich von 15 bis 25°C in Form einer reinen Flüssigkeit vorliegt;
wobei das Verfahren aus den folgenden aufeinanderfolgenden Schritten besteht:
- Zugabe, tropfenweise oder mittels Zerstäuben, des kosmetischen oder pharmazeutischen Mittels im flüssigen Zustand unter Rühren zu den losen Pulverteilchen, so dass es mindestens 25 Gew.-% der Pulverteilchen ausmacht, wobei die Teilchen porös sind und eine spezifische Oberfläche (SSA) über 8 m²/g aufweisen,
- Anhalten der Zugabe, bevor die Pulverteilchen zu agglomerieren beginnen,
- Aufrechterhalten des Rührens für mindestens 5 Minuten, dann
- Gewinnen des erhaltenen, mit dem kosmetischen oder pharmazeutischen Mittel imprägnierten losen Pulvers.

2. Verfahren nach Anspruch 1, wobei das mindestens eine kosmetische oder pharmazeutische Mittel mindestens 40 Gew.-%, bezogen auf das Gewicht der Pulverteilchen, ausmacht.

3. Verfahren nach einem der Ansprüche 1 oder 2, wobei die Teilchen einen volumengemittelten Durchmesser im Bereich von 3 bis 12 µm aufweisen.

4. Verfahren nach einem der vorhergehenden Ansprüche, wobei die Teilchen auf Basis von Polyamid aus Polyamid-, Copolyamid-, Copolyesteramidteilchen und deren Gemischen ausgewählt sind.

5. Verfahren nach einem der vorhergehenden Ansprüche, wobei die Teilchen auf Basis von Polyamid vor der Imprägnierung einen Gehalt an Polyamid 12 in Molprozent im Bereich von 50% bis 100%, vorzugsweise von 80% bis 100% aufweisen.

6. Verfahren nach einem der vorhergehenden Ansprüche, wobei die Pulverteilchen des auf Basis von Polyamid zumindest zum Teil durch anionische Polymerisation, mittels Beimpfen mit einem anorganischen oder organischen Füllstoff, von Lactam(en) und/oder Lacton(en) in Lösung und/oder in Suspension in einer organischen Flüssigkeit erhalten werden.

7. Verfahren nach einem der vorhergehenden Ansprüche, wobei die Teilchen sphäroid sind.

8. Verfahren nach einem der vorhergehenden Ansprüche, wobei die Teilchen Wasser enthalten.

9. Verfahren nach einem der vorhergehenden Ansprüche, wobei das mindestens eine kosmetische oder pharmazeutische Mittel aus feuchtigkeitsspendenden Mitteln, Adstringentien, Antiseptika, entzündungshemmenden Mitteln, Mikrobiziden, Mitteln gegen Cellulitis, Antiseborrhoika, Mattierungsmitteln, Absorptionsmitteln, Antischuppenmitteln, Antifaltenmitteln, Antioxidantien, Mitteln gegen freie Radikale, Glättungsmitteln, Gleitmitteln, Weichmachern, Färbemitteln, Sonnenschutzmitteln, reizlindernden Mitteln, Mitteln gegen Couperose, Wundheilungsmitteln, Abschwellungsmitteln, Regenerationsmitteln, Deodorants, Parfümmitteln, Antitranspirantien, Transpirationshemmern, Enthaarungsmitteln, Stimulatoren des Haar- oder Wimpernwachstums, Depigmentierungsmitteln, Filmbildungsmitteln, Haftmitteln, Fixiermitteln, Pigmentierungsmitteln, Perlglanzmitteln, Selbstbräunungsmitteln, Bräunungsbeschleunigern, Vitaminen und deren Gemischen ausgewählt ist.

10. Verfahren nach Anspruch 9, wobei das mindestens eine kosmetische oder pharmazeutische Mittel aus Erweichungsmitteln, Feuchthaltemitteln, Rückfettungsmitteln und deren Gemischen ausgewählt ist.

11. Verfahren nach Anspruch 9 oder 10, wobei das mindestens eine kosmetische oder pharmazeutische Mittel aus Polyolen, wie Glycerin, Propylenglycol; Glyceriden, wie Triglyceriden; Natriumhyaluronat; Milchsäure; Lipiden; Ceramiden, wie Ceramid-3; und deren Gemischen ausgewählt ist.

12. Verfahren nach einem der vorhergehenden Ansprüche, wobei das mindestens eine kosmetische oder pharmazeutische Mittel aus der Biomassevergärung stammt.

13. Teilchen von losem Pulver auf Basis von Polyamid, die gemäß dem Verfahren nach einem der Ansprüche 1 bis 12 erhalten werden können, wobei die Teilchen porös sind mit einer SSA über 8 m²/g und einem Gehalt von mindestens 25 Gew.-% mindestens eines anderen kosmetischen oder pharmazeutischen Mittels als Wasser, wobei das kosmetische Mittel bei einer Temperatur im Bereich von 15 bis 25°C in Form einer reinen Flüssigkeit vorliegt.

14. Teilchen nach Anspruch 13, wobei das mindestens eine kosmetische oder pharmazeutische Mittel mindestens 40 Gew.-% der Pulverteilchen ausmacht.

15. Teilchen nach einem der Ansprüche 13 oder 14, wobei die Teilchen einen volumengemittelten Durchmesser im Bereich von 3 bis 12 µm aufweisen.

16. Teilchen nach einem der Ansprüche 13 bis 15, wobei die Teilchen auf Basis von Polyamid aus Polyamid-, Copolyamid-, Copolyesteramidteilchen und deren Gemischen ausgewählt sind.

17. Teilchen nach einem der Ansprüche 13 bis 16, wobei die Teilchen auf Basis von Polyamid einen Gehalt an Polyamid 12 in Molprozent von mindestens 50% aufweisen.

18. Teilchen nach einem der Ansprüche 13 bis 17, wobei die Pulverteilchen auf Basis von Polyamid zumindest zum Teil durch anionische Polymerisation, mittels Beimpfen mit einem anorganischen oder organischen Füllstoff, von Lactam(en) und/oder Lacton(en) in Lösung und/oder in Suspension in einer organischen Flüssigkeit erhalten werden.

19. Teilchen nach einem der Ansprüche 13 bis 18, wobei die Teilchen sphäroid sind.

20. Teilchen nach einem der Ansprüche 13 bis 19, wobei das mindestens eine kosmetische oder pharmazeutische Mittel aus feuchtigkeitsspendenden Mitteln, Adstringentien, Antiseptika, entzündungshemmenden Mitteln, Mikrobiziden, Mitteln gegen Cellulitis, Antiseborrhoika, Mattierungsmitteln, Absorptionsmitteln, Antischuppenmitteln, Antifaltenmitteln, Antioxidantien, Mitteln gegen freie Radikale, Glättungsmitteln, Gleitmitteln, Weichmachern, Färbemitteln, Sonnenschutzmitteln, reizlindernden Mitteln, Mitteln gegen Couperose, Wundheilungsmitteln, Abschwellungsmitteln, Regenerationsmitteln, Deodorants, Parfümmitteln, Antitranspirantien, Transpirationshemmern, Enthaarungsmitteln, Stimulatoren des Haar- oder Wimpernwachstums, Depigmentierungsmitteln, Filmbildungsmitteln, Haftmitteln, Fixiermitteln, Pigmentierungsmitteln, Perlglanzmitteln, Selbstbräunungsmitteln, Bräunungsbeschleunigern, Vitaminen und deren Gemischen ausgewählt ist.

21. Teilchen nach Anspruch 20, wobei das mindestens eine kosmetische oder pharmazeutische Mittel aus Erweichungsmitteln, Feuchthaltemitteln, Rückfettungsmitteln und deren Gemischen ausgewählt ist.

22. Teilchen nach Anspruch 20 oder 21, wobei das mindestens eine kosmetische oder pharmazeutische Mittel aus Polyolen, wie Glycerin, Propylenglycol; Glyceriden, wie Triglyceriden; Natriumhyaluronat; Milchsäure; Lipiden; Ceramiden, wie Ceramid-3; und deren Gemischen ausgewählt ist.

23. Teilchen nach einem der Ansprüche 13 bis 22, wobei das mindestens eine kosmetische oder pharmazeutische Mittel aus der Biomassevergärung stammt.

24. Verwendung von Pulverteilchen nach einem der Ansprüche 13 bis 23 in Kosmetik-, Pharmazie- oder Parfümerieprodukten.

25. Kosmetik-, Pharmazie- oder Parfümeriezusammensetzung, **dadurch gekennzeichnet, dass** sie Teilchen nach einem der Ansprüche 13 bis 23 umfasst.

26. Zusammensetzung nach Anspruch 25, wobei die Zusammensetzung ein gefärbtes, farbloses oder transparentes Produkt ist, das aus den folgenden Produkten ausgewählt ist:
- Schminkprodukten für das menschliche Gesicht und den menschlichen Körper, wie Teintgrundierung, getönte Creme, loser oder Kompaktpuder, Lidschatten, Wimperntusche, Eyeliner, Lippenstift;
- Pflegeprodukten für das menschliche Gesicht und den menschlichen Körper, wie Creme, Milch, Lotion, Maske, Peelingprodukt, Reinigungs- und/oder Abschminkprodukte, Deodorants, Antitranspirantien, Transpirationshemmer, Rasierprodukte, Haarentfernungsprodukte;
- Haarprodukten, wie Shampoos, Frisierprodukte, Produkte zur Erhaltung der Frisur, Antischuppenprodukte, Produkte gegen Haarausfall, Produkte gegen Trockenheit der Haare, Haarfärbemittel, Entfärbungsprodukte;
- Parfümerieprodukten, wie Parfüm, Milch, Creme, parfümierter loser oder Kompaktpuder.

## Claims

1. Process for the manufacture of particles of free polyamide-based powder impregnated with at least one cosmetic or pharmaceutical agent, said cosmetic agent being, at a temperature within the range from 15 to 25°C, in the form of a pure liquid;
said process consisting of the following successive stages:
- dropwise addition or addition by spraying of said cosmetic or pharmaceutical agent in the liquid state to particles of free powder with stirring, so that it represents at least 25% by weight of the powder particles, said particles being porous and having an SSA of greater than 8 m²/g,
- stopping the addition before the powder particles begin to agglomerate,
- maintaining the stirring for at least 5 minutes, then
- recovering the free powder obtained impregnated with said cosmetic or pharmaceutical agent.

2. Process according to Claim 1, in which said at least one cosmetic or pharmaceutical agent represents at least 40% by weight of the powder particles.

3. Process according to either one of Claims 1 and 2, in which said particles have a mean diameter by volume within the range extending from 3 to 12 µm.

4. Process according to any one of the preceding claims, in which said polyamide-based particles are chosen from particles of polyamide, copolyamide, copolyesteramide and their mixtures.

5. Process according to any one of the preceding claims, in which said polyamide-based particles comprise, before impregnation, a content, as molar percentage, of polyamide 12 within the range extending from 50% to 100%, preferably from 80% to 100%.

6. Process according to any one of the preceding claims, in which said particles of polyamide-based powder are obtained at least in part by anionic polymerization, by seeding with an inorganic or organic filler, of lactam(s) and/or lactone(s) in solution and/or in suspension in an organic liquid.

7. Process according to any one of the preceding claims, in which said particles are spheroidal.

8. Process according to any one of the preceding claims, in which said particles comprise water.

9. Process according to any one of the preceding claims, in which said at least one cosmetic or pharmaceutical agent is chosen from moisturizing agents, astringents, antiseptic agents, anti-inflammatories, antimicrobial agents, anti-cellulitic agents, antiseborrheic agents, mattifying agents, absorbing agents, antidandruff agents, anti-wrinkle agents, antioxidants, agents for combating free radicals, smoothing agents, lubricants, softeners, colorants, antisun agents, anti-irritants, antirosacea agents, healing agents, decongestants, regenerating agents, deodorants, scenting agents, antiperspirants, depilatory agents, agents which stimulate the growth of head hair or eyelashes, depigmenting agents, film-forming agents, adherent agents, fixing agents, pigmenting agents, pearlescent agents, self-tanning agents, pigmentation photodynamic agents, vitamins and their mixtures.

10. Process according to Claim 9, in which said at least one cosmetic or pharmaceutical agent is chosen from emollients, humectants, relipidizing agents and their mixtures.

11. Process according to Claim 9 or 10, in which said at least one cosmetic or pharmaceutical agent is chosen from polyols, such as glycerol or propylene glycol; glycerides, such as triglycerides; sodium hyaluronate; lactic acid; lipids; ceramides, such as ceramide-3; and their mixtures.

12. Process according to any one of the preceding claims, in which said at least one cosmetic or pharmaceutical agent results from biofermentation.

13. Particles of free polyamide-based powder which are capable of being obtained according to the process of any one of Claims 1 to 12, said particles being porous, having an SSA of greater than 8 m²/g and having a content of at least 25% by weight of at least one cosmetic or pharmaceutical agent other than water, said cosmetic agent being, at a temperature within the range from 15 to 25°C, in the form of a pure liquid.

14. Particle according to Claim 13, in which said at least one cosmetic or pharmaceutical agent represents at least 40% by weight of the powder particles.

15. Particle according to either one of Claims 13 and 14, in which said particles have a mean diameter by volume within the range extending from 3 to 12 µm.

16. Particle according to any one of Claims 13 to 15, in which said polyamide-based particles are chosen from particles of polyamide, copolyamide, copolyesteramide and their mixtures.

17. Particle according to any one of Claims 13 to 16, in which said polyamide-based particles comprise a content, as molar percentage, of polyamide 12 of at least 50%.

18. Particle according to any one of Claims 13 to 17, in which said particles of polyamide-based powder are obtained at least in part by anionic polymerization, by seeding with an inorganic or organic filler, of lactam(s) and/or lactone(s) in solution and/or in suspension in an organic liquid.

19. Particle according to any one of Claims 13 to 18, in which said particles are spheroidal.

20. Particle according to any one of Claims 13 to 19, in which said at least one cosmetic or pharmaceutical agent is chosen from moisturizing agents, astringents, antiseptic agents, anti-inflammatories, antimicrobial agents, anti-cellulitic agents, antiseborrheic agents, mattifying agents, absorbing agents, antidandruff agents, anti-wrinkle agents, antioxidants, agents for combating free radicals, smoothing agents, lubricants, softeners, colorants, antisun agents, anti-irritants, antirosacea agents, healing agents, decongestants, regenerating agents, deodorants, scenting agents, antiperspirants, depilatory agents, agents which stimulate the growth of head hair or eyelashes, depigmenting agents, film-forming agents, adherent agents, fixing agents, pigmenting agents, pearlescent agents, self-tanning agents, pigmentation photodynamic agents, vitamins and their mixtures.

21. Particle according to Claim 20, in which said at least one cosmetic or pharmaceutical agent is chosen from emollients, humectants, relipidizing agents and their mixtures.

22. Particle according to Claims 20 or 21, in which said at least one cosmetic or pharmaceutical agent is chosen from polyols, such as glycerol or propylene glycol; glycerides, such as triglycerides; sodium hyaluronate; lactic acid; lipids; ceramides, such as ceramide-3; and their mixtures.

23. Particle according to any one of Claims 13 to 22, in which said at least one cosmetic or pharmaceutical agent results from biofermentation.

24. Use of powder particles according to any one of Claims 13 to 23 in cosmetic, pharmaceutical or perfumery products.

25. Cosmetic, pharmaceutical or perfumery composition, **characterized in that** it comprises particles as defined in any one of Claims 13 to 23.

26. Composition according to Claim 25, said composition being a colored, colorless or transparent product, chosen from the following products:
- makeup products for the human face and body, such as foundation, tinted cream, loose or compact powder, eyeshadow, mascara, eyeliner or lipstick;
- care products for the human face and body, such as cream, milk, lotion, mask, scrubbing product, cleansing and/or makeup removing products, deodorants, antiperspirants, shaving products or hair-removing products;
- hair products, such as shampoos, products for the shaping of the hair, products for retaining the hairstyle, antidandruff products, products for combating hair loss, products for combating dryness of the hair, hair dyes or bleaching products;
- perfumery products, such as fragrance, milk, cream, or loose or compact scented powder.
